# EUROPEAN PATENT APPLICATION

(11) **EP 4 814 489 A1**
(43) Date of publication of application: **30.09.2026**
(21) Application number: 24894020.7
(22) Date of filing: 08.11.2024
(51) Int. Cl.: G01N 33/543

(54) **IMMUNOCHROMATOGRAPHIC TEST PIECE, IMMUNOCHROMATOGRAPHIC DEVICE, IMMUNOCHROMATOGRAPHIC KIT, AND METHOD FOR DETECTING TEST SUBSTANCE**

(30) Priority: 22.11.2023 JP 2023198004
(71) Applicant: Fujirebio Inc., Tokyo 107-0052 (JP)
(72) Inventor: NOJO MURAKAMI Yukiko, Tokyo 107-0052 (JP); YAMAMOTO Naoki, Tokyo 107-0052 (JP); HIROSE Ryo, Tokyo 107-0052 (JP); HASEGAWA Akira, Tokyo 107-0052 (JP)
(74) Representative: Müller-Boré & Partner Patentanwälte PartG mbB
(86) International application number: PCT/JP2024/039782
(87) International publication number: WO 2025/110028

(57) **Abstract**

An immunochromatographic test piece for use in detecting a target substance in a sample containing blood cells by immunochromatography, comprising:
a matrix for developing a developing solution;
a blood cell removal pad including an addition part of the sample, laminated on the matrix; and
a waterproof layer laminated between the matrix and the blood cell removal pad,
wherein the blood cell removal pad has a protruding portion protruding onto the matrix further in a development direction of the developing solution than the waterproof layer.

## Description

### [Technical Field]

The present invention relates to an immunochromatographic test piece, an immunochromatographic device (apparatus), an immunochromatographic kit, and a method for detecting test substance (target substance); and more specifically relates to an immunochromatographic test piece, an immunochromatographic apparatus comprising the same, an immunochromatographic kit including at least any of these, and a method for detecting a target substance in a sample containing blood cells using at least any of these.

### [Background Art]

In order to determine positive/negative for infectious diseases or identify the causes thereof, it is necessary to detect pathogens such as bacteria and viruses from a sample collected from a subject. Also, detecting a substance serving as a marker of a specific disease from a sample such as blood for positive/negative determination of the disease is also known. As a method for detecting such a target substance such as a pathogen or a marker, an immunoassay utilizing an antigen-antibody reaction is known.

Among the immunoassays, an immunochromatographic method is known as a simple and rapid detection method. As the immunochromatographic method, for example, a method is cited in which a sample is developed on an immunochromatographic test piece using, as an immunochromatographic medium, a porous membrane or the like on which an antibody or antigen or the like capable of binding to a target substance in the sample is immobilized; in the process of the development, a complex of a labeling body capable of specifically binding to the target substance (an antibody or antigen or the like capable of specifically binding to the target substance and labeled with a labeling substance) and the target substance is formed; the complex is captured indirectly or directly by the antigen or antibody immobilized on the porous membrane or the like; and the target substance is detected by a signal such as color development by the labeling substance. Such a method allows detection of the target substance in the sample simply by applying or dropping the sample onto the immunochromatographic test piece and developing the sample, the complex, or the like on the immunochromatographic medium by capillary action; therefore, the operation is simple. Also, since detection of the target substance is possible in a short time and visual determination is also possible, it is widely utilized in research tests and clinical examinations.

However, when the sample is a sample containing blood cells such as a whole blood specimen, clogging of the immunochromatographic medium is caused by the blood cells, or if red blood cells rupture, coloration by the hemoglobin makes it difficult to visually confirm the signal by the labeling substance easily; thus, a problem arises. Therefore, when the sample contains blood cells, it is necessary to remove the blood cells in advance; in the immunochromatographic test piece, for example, a method of installing a member having a blood cell removal function on the test piece itself is used.

For example, Japanese Unexamined Patent Application Publication No. 2002-350428 (Patent Literature 1) describes that, for the purpose of suppressing hemolysis of red blood cells, a fiber-containing layer for holding red blood cells and separating plasma or serum from whole blood is composed of glass fibers coated with propanol with a butoxy group and/or acrylamide.

Also, as what aims to eliminate the influence of blood cells by the structure of the test piece, for example, in Japanese Unexamined Patent Application Publication No. 2013-181870 (Patent Literature 2), an immunochromatographic test strip is described which comprises a chromatography medium having a determination part supported on an impermeable base material and a specimen treatment part to which a sample is added upstream thereof , and has a structure in which the specimen treatment part is formed by laminating a sample pad to which a sample is added, a blood cell separation membrane for separating blood cells, and a conjugate pad containing a labeling substance while shifting them partially in order.

Furthermore, for example, International Application Japanese-Phase Publication No. 2011-522228 (Patent Literature 3) describes a test strip used for a method of determining a blood type based on whether aggregated red blood cells remain in a reaction spot after dropping a blood specimen onto a test strip on which each anti-N blood group antibody is coated and immobilized and dropping a washing liquid onto one end of the test strip, in which a washing pad, a sample application pad, a buffer pad, and a water absorption pad are sequentially pasted on a lining, and a waterproof tape is pasted on the upper surface of an overlapping edge portion of the washing pad and the sample application pad for the purpose of preventing the washing liquid from spreading from the surface of the sample application pad.

Also, for example, International Application Japanese-Phase Publication No. 2017-509889 (Patent Literature 4) describes a liquid guiding element for a lateral flow test device and that the amount of sample is small in the case of a blood specimen; and as a configuration improved so that the necessary volume of the sample is reduced, a configuration is described in which each member constituting the liquid guiding element is each provided with a backing layer, and the backing layer is oriented so as to be installed on the upper surface or the lower surface centering on the liquid guiding element.

### [Citation List]

### [Patent Literature]

[PTL 1] Japanese Unexamined Patent Application Publication No. 2002-350428
[PTL 2] Japanese Unexamined Patent Application Publication No. 2013-181870
[PTL 3] International Application Japanese-Phase Publication No. 2011-522228
[PTL 4] International Application Japanese-Phase Publication No. 2017-509889

### [Summary of Invention]

### [Technical Problem]

Conventionally, in order to remove blood cells from a sample containing blood cells on an immunochromatographic test piece, as described above, it has been necessary to use a specific base material for a member having a blood cell removal function, or to dispose a sample addition part or a member having a blood cell removal function upstream of a strip-shaped test piece so that blood cells are removed in the process of development. Also, for example, when an enzyme is used as a labeling substance, a signal is detected by a reaction between the enzyme and a substrate; however, for detection with high accuracy, it is preferable that the reaction between the enzyme and the substrate occurs after a complex of a labeling body containing the enzyme and a target substance is captured by an antibody or antigen for capture. However, in the conventional structure in which the addition part of the sample is upstream of the test piece, if development of the complex and the substrate is carried out in a single operation, the capture of the complex and the enzyme-substrate reaction cannot be carried out with a time difference; thus, there has been a problem that accuracy decreases when applied to enzyme immunoassay.

The present invention has been made in view of the above problems, and aims to provide an immunochromatographic test piece capable of easily detecting a target substance in a sample containing blood cells such as a whole blood specimen and also allowing enzyme immunoassay to be performed with high accuracy, an immunochromatographic apparatus comprising the same, an immunochromatographic kit including at least any of these, and a method for detecting a target substance in a sample containing blood cells using at least any of these.

### [Solution to Problem]

The present inventors have conducted intensive studies on a test piece used for detecting a target substance in a sample containing blood cells by immunochromatography in order to solve the above problems. As a result, they have found that, by adopting a structure in which a waterproof layer separating a matrix, which is an immunochromatographic medium for developing a developing solution, and a blood cell removal pad having a blood cell removal function is sandwiched therebetween, and the blood cell removal pad protrudes slightly further in a development direction of the developing solution than the waterproof layer, and the matrix and the blood cell removal pad are in contact only at this protruding portion, and by adding a sample to the blood cell removal pad, despite such an extremely simple structure, blood cells are removed from the sample before contacting the developing solution developing in the matrix, migration of blood cells to the matrixis sufficiently suppressed, and the target substance can be easily detected. Moreover, according to this structure, it is possible to install an addition part of the sample and a developing solution supply part for supplying the developing solution separately at different positions. Therefore, for example, they have found that by holding a labeling body in the addition part and installing it in the center of the test piece, while installing a developing solution supply part for supplying the developing solution and a substrate zone containing a substrate to be eluted into the developing solution upstream of the test piece, it is possible to react the enzyme contained in the labeling body with the substrate that has arrived with a delay after causing the complex of the target substance and the labeling body to be captured by an antibody or antigen (capturing body) for capture downstream of the addition part first; thus, highly accurate enzyme immunoassay can also be easily carried out, leading to the completion of the present invention.

That is, the present invention relates to an immunochromatographic test piece, an immunochromatographic apparatus comprising the same, an immunochromatographic kit including these, and a target substance detection method using these, and more specifically provides the following.
[1] An immunochromatographic test piece for use in detecting a target substance in a sample containing blood cells by immunochromatography, comprising:
   a matrix for developing a developing solution;
   a blood cell removal pad including an addition part of the sample, laminated on the matrix; and
   a waterproof layer laminated between the matrix and the blood cell removal pad,
   wherein the blood cell removal pad has a protruding portion protruding onto the matrix further in a development direction of the developing solution than the waterproof layer.
[2] The immunochromatographic test piece according to [1], wherein a length of the protruding portion of the blood cell removal pad is from 1 mm to 4 mm.
[3] The immunochromatographic test piece according to [1] or [2], wherein the blood cell removal pad is a glass fiber membrane.
[4] The immunochromatographic test piece according to any one of [1] to [3], comprising a capturing body immobilization part in which a capturing body capable of capturing the target substance is immobilized, downstream of the blood cell removal pad on the matrix.
[5] The immunochromatographic test piece according to any one of [1] to [4], comprising a labeling body-containing part that holds, in an elutable manner, a labeling body in which a labeling substance is bound to an antibody or antigen capable of binding to the target substance.
[6] The immunochromatographic test piece according to [5], wherein the labeling substance is an enzyme.
[7] The immunochromatographic test piece according to [6], wherein the developing solution contains a substrate of the enzyme, or the immunochromatographic test piece comprises a substrate zone that holds a substrate of the enzyme in an elutable manner upstream of the capturing body immobilization part on the matrix, or comprises an addition part of a substrate of the enzyme on the matrix.
[8] The immunochromatographic test piece according to any one of [5] to [7], wherein a labeling body-containing pad including the labeling body-containing part is laminated on the blood cell removal pad, or the blood cell removal pad includes the labeling body-containing part.
[9] An immunochromatographic apparatus used for detecting a target substance in a sample containing blood cells by immunochromatography, comprising the immunochromatographic test piece according to any one of [1] to [8].
[10] An immunochromatographic kit used for detecting a target substance in a sample containing blood cells by immunochromatography, including the immunochromatographic test piece according to any one of [1] to [8] or the immunochromatographic apparatus according to [9].
[11] A method for detecting a target substance in a sample containing blood cells by immunochromatography, comprising the steps of:
   in an immunochromatographic test piece comprising: a matrix for developing a developing solution; a blood cell removal pad including an addition part of the sample, laminated on the matrix; and a waterproof layer laminated between the matrix and the blood cell removal pad, wherein the blood cell removal pad has a protruding portion protruding onto the matrix further in a development direction of the developing solution than the waterproof layer,
   adding the sample to the addition part; and
   causing the matrix to develop a developing solution,
   wherein the sample and the developing solution are brought into contact at the protruding portion of the blood cell removal pad.

Note that although the reason why the above object is achieved by the configuration of the present invention is not necessarily certain, the present inventors infer as follows. That is, in the immunochromatographic test piece of the present invention, when a sample containing blood cells is added to the addition part of the blood cell removal pad from an upper surface, the sample does not migrate in a vertical direction to the matrix under the blood cell removal pad because the waterproof layer exists on a lower surface side of the blood cell removal pad. Also, regarding the blood cell removal pad and the waterproof layer, since the blood cell removal pad has a structure having a protruding portion protruding onto the matrix further in the development direction of the developing solution than the waterproof layer, the added sample moves in the development direction of the developing solution moving in the matrix and in a direction parallel thereto. Therefore, while moving in this blood cell removal pad, the sample is filtered and blood cells are removed, and only the sample from which blood cells have been removed migrates to the matrix from the protruding portion at a tip of the blood cell removal pad. Therefore, the present inventors infer that migration of blood cells to the matrix can be simply and efficiently suppressed, and detection of the target substance can be carried out easily and with high accuracy.

### [Advantageous Effects of Invention]

According to the present invention, it becomes possible to provide an immunochromatographic test piece capable of easily detecting a target substance in a sample containing blood cells such as a whole blood specimen and also allowing enzyme immunoassay to be performed with high accuracy, an immunochromatographic apparatus comprising the same, an immunochromatographic kit including at least any of these, and a method for detecting a target substance in a sample containing blood cells using at least any of these.

### [Brief Description of Drawings]

[Fig. 1] Fig. 1 is a schematic cross-sectional view showing one embodiment (a: immunochromatographic test piece 101, b: immunochromatographic test piece 102) of the immunochromatographic test piece of the present invention.
[Fig. 2] Fig. 2 is a schematic plan view showing one embodiment of the immunochromatographic test piece (immunochromatographic test piece 103) of the present invention.
[Fig. 3] Fig. 3 is a schematic cross-sectional view of Fig. 2 showing one embodiment of the immunochromatographic test piece (immunochromatographic test piece 103) of the present invention.
[Fig. 4] Fig. 4 is a schematic cross-sectional view showing one embodiment of a main part of the immunochromatographic apparatus (immunochromatographic apparatus 201) of the present invention.
[Fig. 5] Fig. 5 is a schematic plan view showing one embodiment of the immunochromatographic apparatus (immunochromatographic apparatus 202) of the present invention.
[Fig. 6] Fig. 6 is a schematic cross-sectional view of Fig. 5 showing one embodiment of the immunochromatographic apparatus (immunochromatographic apparatus 202) of the present invention.
[Fig. 7] Fig. 7 is a view showing the appearance of a determination window (detection zone 8 and development confirmation zone 11) at the time of visual confirmation when a detection test of target substances (antigen A and antibody B) was performed using each immunochromatographic apparatus produced in Examples 1 and 3.
[Fig. 8] Fig. 8 is a view showing the appearance of the determination window (detection zone 8 and development confirmation zone 11) and evaluation results thereof when a comparative test of each immunochromatographic apparatus produced in Examples 1 to 3 (upper row) and Comparative Examples 1 to 3 (lower row) was performed with each dropping amount for a healthy person whole blood specimen (negative specimen).
[Fig. 9] Fig. 9 is a conceptual diagram showing positions a to c of each labeling body-containing part in the produced immunochromatographic apparatus in "(4-1) Production of Immunochromatographic Apparatus" of "(4) Study of Immunochromatographic Apparatus."
[Fig. 10] Fig. 10 is a view showing the appearance of the determination window (detection zone 8 and development confirmation zone 11) and evaluation results at the time of visual confirmation when a detection test of a target substance (antigen C) was performed using each immunochromatographic apparatus produced in Examples 1-1, 1-2, 3-1, and 3-2.

### [Description of Embodiments]

Hereinafter, the present invention will be described in detail with respect to preferred embodiments thereof by giving examples with reference to drawings in some cases , but the present invention is not limited thereto. Note that in the following description and drawings, the same or corresponding elements are denoted by the same reference signs, and overlapping description is omitted.

### <Immunochromatographic Test Piece, Immunochromatographic Apparatus>

The present invention is a test piece used for detecting a target substance in a sample containing blood cells by immunochromatography, and provides an immunochromatographic test piece comprising:
a matrix for developing a developing solution;
a blood cell removal pad including an addition part of the sample, laminated on the matrix; and
a waterproof layer laminated between the matrix and the blood cell removal pad,
wherein the blood cell removal pad has a protruding portion protruding onto the matrix further in a development direction of the developing solution than the waterproof layer. Also, the present invention provides an immunochromatographic apparatus comprising the immunochromatographic test piece of the present invention described above.

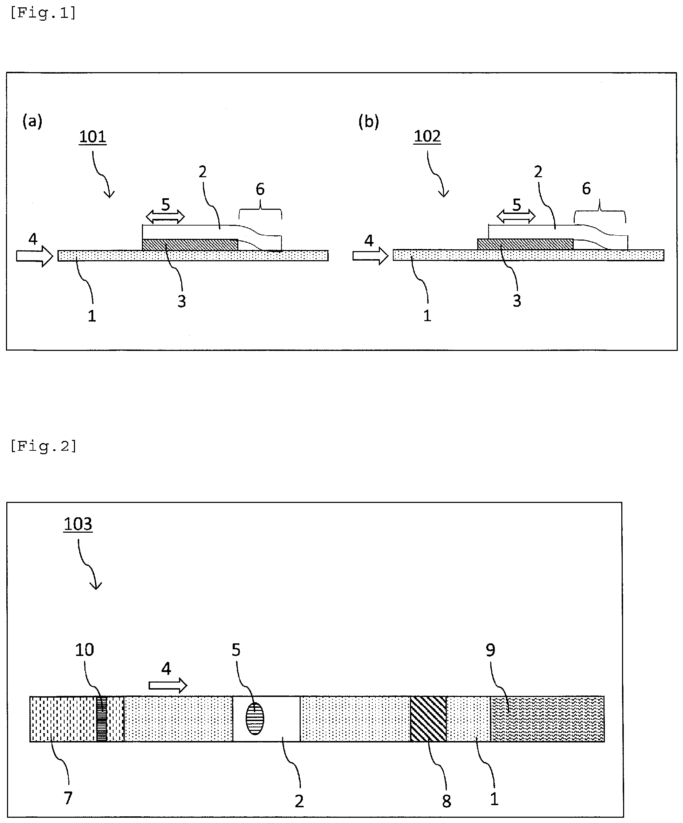

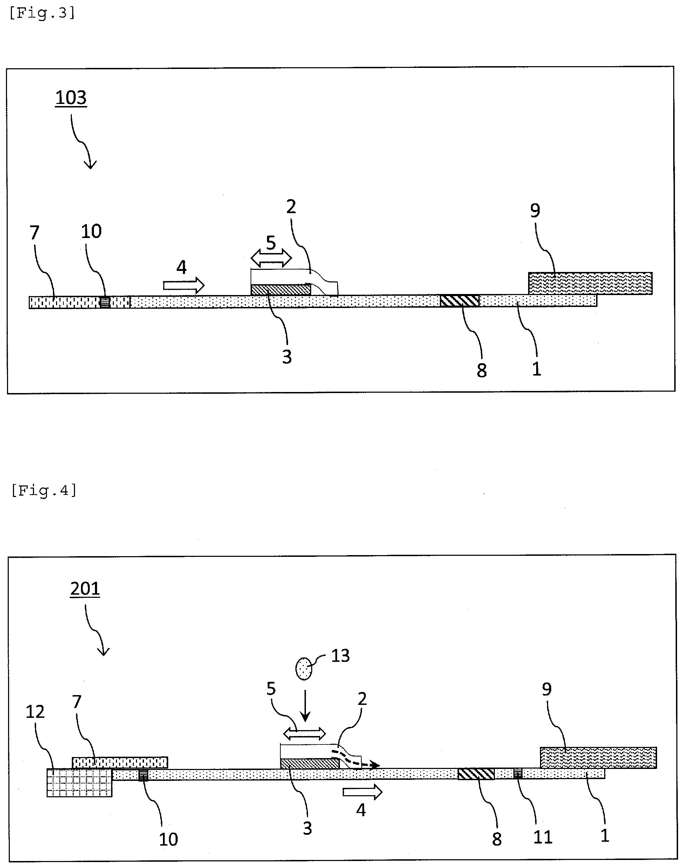

Figs. 1 to 3 are schematic cross-sectional views or plan views showing one embodiment of the immunochromatographic test piece of the present invention, and Figs. 4 to 6 are schematic cross-sectional views or plan views showing one embodiment of the immunochromatographic apparatus of the present invention. In the following description, the left side toward Figs. 1 to 6 (upstream side in the development direction 4 of the developing solution) is expressed as "upstream" in the immunochromatographic test piece, and the opposite side thereof is expressed as "downstream." Note that in Figs. 1 to 6, a strip for lateral flow immunochromatography is described as an example as one embodiment of the immunochromatographic test piece, but the immunochromatographic test piece of the present invention is not limited to the embodiment shown in Figs. 1 to 6.

### (Sample)

In the present invention, a sample to be a target of detection is a sample containing blood cells. However, this does not deny targeting a sample not containing blood cells. Examples of the blood cells include red blood cells, white blood cells, and platelets, and it is preferable that the sample contains at least red blood cells. Such a sample is not particularly limited as long as it contains the blood cells, and may be, for example, a suspension of blood cells or the like, but is usually a whole blood specimen. As the whole blood specimen, for example, a whole blood specimen collected from a human and a mammal other than a human (preferably a human) can be cited. Also, the sample may be one obtained by diluting or suspending the whole blood specimen with a diluent or the like as necessary; examples of the diluent include buffer solutions (phosphate buffer solution, Tris buffer solution, Good's buffer solution, borate buffer solution, etc.), and one kind or two or more kinds among surfactants, blood coagulation inhibitors, and the like may be contained as long as each antigen-antibody reaction is not inhibited.

### (Target Substance)

In the present invention, the "target substance" to be detected is not particularly limited, and examples thereof include those that can be contained in the whole blood specimen; pathogens such as bacteria, protists, fungi, and viruses, and substances derived therefrom such as nucleic acids, sugar chains, and proteins, as well as antibodies against these; and substances serving as markers of specific diseases (proteins, polysaccharides, nucleic acids, etc.) are cited.

### (Detection)

In the present invention, the target substance is detected by immunochromatography utilizing an antigen-antibody reaction between an antibody or antigen capable of binding to the target substance (in the present specification, occasionally referred to as a "detection body") and the target substance. When the target substance is an antibody, the detection body can be an antigen thereof (including a substance showing the same property as the antigen in immunoassay (for example, a partial peptide containing an epitope of the antigen, an analogous structure (analog), a derivative, a modified body, etc.)) or an antibody capable of binding to the antibody; and when the target substance is other than an antibody, the detection body can be an antibody capable of binding to the target substance.

In the present invention, the "antibody" includes, in addition to a complete antibody, antibody fragments (for example, Fab, Fab', F(ab')₂, Fv, single-chain antibody, diabody, etc.) and low-molecular-weight antibodies in which variable regions of an antibody are combined. When the detection body is an antibody, the antibody may be a polyclonal antibody or a monoclonal antibody. Such an antibody can be produced by appropriately adopting or improving a conventionally known production method or a method according thereto depending on the desired target substance, and one generally distributed may be appropriately used.

In the present invention, the "detection of a target substance" includes detection for confirming the presence or absence of the target substance, and quantitation or semi-quantitation of the amount of the target substance. In an immunochromatographic test piece, usually, detection of a target substance is performed by using a labeling body in which a labeling substance is bound to the detection body, binding this labeling body to the target substance via the detection body, detecting a signal derived from the labeling substance contained in the formed complex, and quantifying the intensity thereof as necessary, which is preferable in the present invention. Also, as necessary, success or failure of detection can be determined, for example, by detecting a signal of a development confirmation zone described below. In the present invention, the "signal" includes coloration (color development), decoloration, quenching, reflected light, luminescence, fluorescence, radiation by a radioisotope, and the like, and includes those confirmable by a detection method/apparatus according to the type of the signal in addition to those confirmable with the naked eye.

As the labeling substance, those used as labeling substances in known immunoassay methods or methods according there to can be used without particular limitation. For example, enzymes such as horseradish peroxidase (HRP), alkaline phosphatase (ALP), beta-galactosidase (beta-gal), glucose oxidase, and luciferase; radioisotopes such as iodine, tritium, and carbon; luminescent substances such as acridinium derivatives; fluorescent substances such as europium; fluorescent proteins such as allophycocyanin (APC) and phycoerythrin (R-PE); low-molecular-weight labeling substances such as fluorescein isothiocyanate (FITC) and rhodamine isothiocyanate (RITC); gold particles; latex; dinitrophenyl (DNP); and digoxigenin (DIG) are cited, and it may be one kind among these or a combination of two or more kinds. Among these , the labeling substance is preferably an enzyme from the viewpoint that the immunochromatographic test piece of the present invention can be suitably applied. When an enzyme is used as the labeling substance, by using a chromogenic substrate, a fluorescent substrate, a chemiluminescent substrate, or the like as a substrate of the enzyme, various signals generated by the reaction between the enzyme and the substrate can be detected.

As the labeling body, the detection body and the labeling substance may be directly bound or indirectly bound, and it may further contain a water-soluble carrier or the like supporting the detection body and the labeling substance. Examples of the water-soluble carrier include saccharides such as dextran, aminodextran, Ficoll (trade name), dextrin, agarose, pullulan, various celluloses (for example, hemicellulose and lignin), chitin, chitosan, and modified bodies thereof (for example, hydrazinodextran); proteins such as beta-galactosidase and thyroglobulin; polypeptides; DNA; hemocyanin; and amino acids such as polylysine, and it may be one kind among these or a combination of two or more kinds.

For binding between the detection body and the labeling substance, a conventionally known method or a method according thereto can be appropriately adopted; the detection body and the labeling substance may be directly bound by an active group or the like, or may be indirectly bound via an oligopeptide, a linker, or the like, or a biotin-avidin system (for example, utilizing interaction between biotin and avidin by biotinylating the detection body, causing an avidinylated labeling substance to act thereon, and binding the labeling substance to the detection body) may be utilized. The amounts of the detection body, the labeling substance, and the water-soluble carrier as necessary in the labeling body can be appropriately adjusted. Also, as such a labeling body, one generally distributed may be appropriately used.

Also, in the detection of the target substance by the immunochromatographic test piece, the labeling substance may not be bound to the detection body, but a secondary labeling body may be bound to the detection body and this may be detected. Here, the "secondary labeling body" is a labeling body capable of binding to the detection body and provided with the labeling substance. Examples of such a secondary labeling body include an antibody (secondary antibody or the like) to which the labeling substance is bound and which is capable of binding to the detection body, and Protein G, Protein A, or the like to which the labeling substance is bound.

### (Matrix)

The immunochromatographic test piece of the present invention comprises a matrix (matrix 1 in Figs. 1 to 4) for developing a developing solution. The matrix according to the present invention is an insoluble carrier functioning as an immunochromatographic medium and a stationary phase. The matrix supports a blood cell removal pad and a waterproof layer described below as a main body of the immunochromatographic test piece, and also serves as an antibody-containing membrane including a capturing body immobilization part, a labeling body-containing part, a development confirmation zone, and the like described below, and/or a substrate-containing membrane including a substrate zone and the like described below, as necessary.

The matrix according to the present invention is not particularly limited, and those conventionally used as immunochromatographic media can be appropriately used, but a porous membrane is preferable; examples thereof include a nitrocellulose membrane, a nitrocellulose mixed ester membrane, a cellulose membrane, an acetylcellulose membrane, a polysulfone membrane, a polyethersulfone membrane, a nylon membrane, glass fiber, a nonwoven fabric, a cloth, and a laminate of two or more kinds among these, and among these, a nitrocellulose membrane is preferable. The moving speed of the developing solution developed in such a matrix can be changed by the material, size, pore diameter, distribution, and the like of the porous membrane, and can be appropriately adjusted according to the amount of the sample, the concentration of the target substance, the purpose of detection, and the like.

The shape of the matrix, that is, the shape of the immunochromatographic test piece of the present invention is not particularly limited, and examples thereof include a form in which the developing solution is developed concentrically as a circular shape, and a form in which the developing solution is developed with one end as upstream and the other end as downstream as a strip (elongated piece) shape, but the strip shape is preferable. The size of the matrix is not particularly limited, but can be, for example, a strip (elongated piece) shape with a width of about 3 mm to 10 mm and a length of about 30 mm to 100 mm. The thickness of the matrix is also not particularly limited, but examples thereof include a range of 100 µm to 1 mm. Note that in the present specification, a distance in the development direction of the developing solution is expressed as "length," and when the immunochromatographic test piece is in a strip shape, the size of a short side of the matrix or a side parallel thereto is expressed as "width," and the size of a long side of the matrix or a side parallel thereto is expressed as "length."

### (Blood Cell Removal Pad)

The immunochromatographic test piece of the present invention comprises a blood cell removal pad (blood cell removal pad 2 in Figs. 1 to 4) on the matrix. The blood cell removal pad according to the present invention has a function of filtering and removing blood cells from the sample.

The blood cell removal pad according to the present invention is not particularly limited as long as it can filter blood cells from the sample, but is preferably a porous membrane; examples thereof include a nitrocellulose membrane, a nitrocellulose mixed ester membrane, a cellulose membrane, an acetylcellulose membrane, a polysulfone membrane, a polyethersulfone membrane, a nylon membrane, a glass fiber membrane, a nonwoven fabric, and a cloth, and among these, a glass fiber membrane is preferable.

As conditions under which the blood cell removal pad can filter and remove blood cells from the sample, for example, the particle retention capacity is preferably from 2 µm to 3.5 µm, more preferably from 2.2 µm to 3 µm, and further preferably from 2.2 µm to 2.8 µm. In the present invention, the "particle retention capacity" indicates an average particle diameter (µm) of particles (blood cells) 98% or more of which are retained in the porous membrane, and can be measured by, for example, flow cytometry or the like.

Also, as the blood cell removal pad according to the present invention, the flow rate is preferably from 20 s/4 cm to 50 s/4 cm, more preferably from 20 s/4 cm to 46 s/4 cm, and further preferably from 25 s/4 cm to 46 s/4 cm. In the present invention, the "flow rate" of the porous membrane indicates a speed at which water passes through the inside of the porous membrane from a lower surface toward an upper surface direction (time for passing through the porous membrane having a thickness of 4 cm: s/4 cm) under atmospheric pressure at an air temperature of 20°C and a humidity of 50%, and is preferably an average value of multiple measurements.

Furthermore, as the blood cell removal pad according to the present invention, the water absorption amount is also preferably from 30 mg/cm² to 120 mg/cm², and more preferably from 40 mg/cm² to 120 mg/cm². In the present invention, the "water absorption amount" of the porous membrane indicates an amount of water absorbed in 1 cm² of the porous membrane under atmospheric pressure at an air temperature of 20°C and a humidity of 50%, and can be obtained by measuring the mass of the porous membrane before and after water absorption and taking the difference (preferably an average value of multiple measurements) .

The size of the blood cell removal pad according to the present invention can be appropriately adjusted according to the amount of the sample, the concentration of the target substance, the purpose of detection, and the like; when the matrix is in a strip shape, for example, the width is preferably from 3 mm to 10 mm and the length is from 10 mm to 30 mm, and more preferably the width is from 3 mm to 10 mm and the length is from 10 mm to 20 mm.

Also, the thickness of the blood cell removal pad is preferably from 240 µm to 1000 µm, more preferably from 450 µm to 950 µm, and further preferably from 480 µm to 930 µm. In the present invention, the "thickness" of the porous membrane is measured by a caliper or the like under atmospheric pressure at an air temperature of 20°C and a humidity of 50%, and is preferably an average value of multiple measurements. The thickness of the blood cell removal pad can be appropriately adjusted by the addition amount of the sample or the like; for example, when the addition amount of the sample per unit area is from 20 µL/cm² to 60 µL/cm², it is preferably from 240 µm to 600 µm; when the addition amount of the sample is from 60 µL/cm² to 90 µL/cm², it is preferably from 600 µm to 900 µm; and when the addition amount of the sample is from 90 µL/cm² to 110 µL/cm², it is preferably from 900 µm to 1000 µm.

### (Addition Part)

The blood cell removal pad according to the present invention includes an addition part (addition part 5 in Figs. 1 to 6) of the sample as a site for applying or dropping the sample onto the immunochromatographic test piece.

In the blood cell removal pad, the position of the addition part is an upper surface of the blood cell removal pad. Such an addition part is preferably on an upstream side in the blood cell removal pad, more preferably within the blood cell removal pad and within 15 mm from an upstream end of the blood cell removal pad, further preferably within an area of 50% on the upstream end side among the total area of the blood cell removal pad, and even more preferably within an area of 32%.

The size of the addition part according to the present invention is not particularly limited, but when the matrix is in a strip shape, examples thereof include a range of a width of 1 mm to 10 mm and a length of 3 mm to 15 mm.

### (Waterproof Layer)

The immunochromatographic test piece of the present invention comprises a waterproof layer (waterproof layer 3 in Figs. 1 to 4) between the matrix and the blood cell removal pad. The waterproof layer according to the present invention has a function of blocking migration of the sample and the developing solution between the matrix and the blood cell removal pad and a function of guiding the sample existing on the waterproof layer downstream of the blood cell removal pad.

The waterproof layer according to the present invention is not particularly limited as long as it is a base material that does not permeate the sample and the developing solution; examples thereof include films made of polyester, polyethylene, polyethylene terephthalate, polypropylene, and a laminate of two or more kinds among these, and among these, a polyester film is preferable.

As for the size of the waterproof layer according to the present invention, it is sufficient if the portion other than the protruding portion described below is the same as or larger than the blood cell removal pad so that migration of the sample and the developing solution between the matrix and the blood cell removal pad can be blocked in portions other than the protruding portion described below, that is, so that the blood cell removal pad and the matrix do not come into direct contact in portions other than the protruding portion, and it can be appropriately adjusted according to the size of the blood cell removal pad; however, when the matrix is in a strip shape, for example, the width is preferably from 3 mm to 10 mm and the length is from 8 mm to 28 mm, and more preferably the width is from 3 mm to 10 mm and the length is from 8 mm to 18 mm. Also, the thickness of the waterproof layer is preferably from 0.0005 mm to 1 mm, and more preferably from 0.0005 mm to 0.1 mm.

### (Protruding Portion)

In the immunochromatographic test piece of the present invention, the blood cell removal pad and the waterproof layer are laminated such that the blood cell removal pad protrudes onto the matrix further in the development direction of the developing solution than the waterproof layer, that is, the blood cell removal pad has a protruding portion (protruding portion 6 in Fig. 1) protruding onto the matrix further in the development direction of the developing solution than the waterproof layer.

Fig. 1 shows schematic cross-sectional views of an immunochromatographic test piece 101 and an immunochromatographic test piece 102 as one aspect of the immunochromatographic test piece of the present invention. In the immunochromatographic test piece of the present invention, regarding the blood cell removal pad 2 and the waterproof layer 3, it is sufficient if they are the same size other than the protruding portion 6 or the waterproof layer 3 is larger (that is, if the blood cell removal pad 2 and the matrix 1 are isolated by the waterproof layer 3 other than the protruding portion 6); for example, as shown in Fig. 1(a), the upstream ends (upstream in the development direction 4 of the developing solution) may overlap, or as shown in Fig. 1(b), the upstream ends (upstream in the development direction 4 of the developing solution) may be shifted. As shown in Fig. 1, it is preferable that at least a part of the protruding portion 6 is in direct contact with the matrix 1, but it does not have to be completely in contact as long as the sample is movable by surface tension of the liquid or the like.

The length of the protruding portion is preferably from 1 mm to 4 mm, and more preferably from 2 mm to 4 mm. Also, the length of the protruding portion is preferably such a length that the area of the protruding portion accounts for 13% to 34% on the downstream end side among the total area of the blood cell removal pad, and more preferably a length accounting for 13% to 20%. Note that the length of the protruding portion indicates a length not considering curvature of the blood cell removal pad, that is, a length from a downstream end of the waterproof layer, defined as 0 mm, to a downstream end of the blood cell removal pad protruding therefrom when the immunochromatographic test piece is turned upside down and placed on a plane surface in the order of the blood cell removal pad and the waterproof layer from the bottom. Also, when the matrix is in a strip shape, the width of the protruding portion is preferably from 3 mm to 10 mm. Note that in Figs. 1 to 4, the shape of the protruding portion 6 is a rectangle along the shape of the matrix, which is preferable, but the shape of the protruding portion according to the present invention is not limited thereto, and may be an arbitrary shape such as a semicircle or a semi-polygon. In this case, the width of the protruding portion indicates the longest length in the vertical direction from the downstream end of the waterproof layer, defined as 0 mm, to the downstream end of the blood cell removal pad protruding therefrom.

### (Capturing Body Immobilization Part)

It is preferable that the immunochromatographic test piece of the present invention comprises a capturing body immobilization part (capturing body immobilization part 8 in Figs. 2 to 6) in which a capturing body capable of capturing the target substance is immobilized to the matrix, downstream of the blood cell removal pad on the matrix. The capturing body immobilization part functions as a detection zone of the immunochromatographic test piece of the present invention. Thereby, for example, detection of the target substance captured by the capturing body can be performed by a signal according to the type of the labeling substance by causing the capturing body to capture the target substance in the capturing body immobilization part and then reacting it with the labeling body recognizing the target substance or a complex of the capturing body and the target substance.

As another aspect, more preferably, first, a complex of the target substance and the labeling body is formed, then the complex is captured by the capturing body in the capturing body immobilization part to form a complex of capturing body-target substance-labeling body, and the complex captured by the capturing body, that is, the target substance is detected by a signal according to the type of the labeling substance. In this case, when the complex of the target substance and the labeling body moves to the capturing body immobilization part, the complex is captured by the capturing body immobilized to the capturing body immobilization part, and the labeling substance constituting the complex accumulates in the capturing body immobilization part. As a result, since the capturing body immobilization part shows a signal due to accumulation of the labeling substance, the target substance in the sample can be detected by detecting this.

In the present invention, the "capturing body capable of capturing the target substance" is a substance capable of binding to the target substance or the complex of the target substance and the labeling body, and capable of capturing the target substance or the complex. Such a capturing body typically includes an antibody or antigen capable of binding to the target substance; or an antibody capable of specifically binding to a binding part between the target substance and the antibody or antigen contained in the labeling body. The antibody or antigen contained in such a capturing body may be the same as or different from the antibody or antigen cited as the detection body in the labeling body, but depending on a recognized site of the target substance, it is preferable that they do not compete with each other in binding to the target substance.

Note that in Figs. 2 to 6, the capturing body immobilization part 8 is formed in a line shape, but the shape of the capturing body immobilization part according to the present invention is not limited thereto, and may be an arbitrary shape such as a circle or a polygon. Among these shapes, the shape of the capturing body immobilization part according to the present invention is preferably a line shape, and when the matrix is in a strip shape, for example, a line shape with a width of 3 mm to 10 mm and a length of 0.5 mm to 3 mm is more preferable.

Also, a plurality of capturing body immobilization parts according to the present invention may be provided depending on the type of the target substance and the detection method. For example, when a labeling body A containing an antibody 'a' capable of binding to a target substance 'a' and a labeling body B containing an antibody 'b' capable of binding to a target substance 'b' are used as the labeling body, correspondingly, a capturing body immobilization part A to which an antibody capable of binding to the target substance 'a' is immobilized and a capturing body immobilization part B to which an antibody capable of binding to the target substance 'b' is immobilized may be provided.

As a method for immobilizing the capturing body to the capturing body immobilization part according to the present invention, there is no particular limitation, and a conventionally known method or a method according thereto can be appropriately adopted; for example, a method of binding to the matrix by a physical adsorption method or a covalent bond is cited, and blocking may be performed as necessary. The amount of the capturing body to be immobilized to the capturing body immobilization part according to the present invention can be appropriately adjusted by the amount of the sample, the type of the detection body contained in the labeling body, sensitivity of the capturing body to the target substance, the purpose of detection, and the like.

### (Labeling Body-Containing Part)

In the present invention, the sample may be mixed with the labeling body in advance and then added to the addition part; however, it is preferable that the immunochromatographic test piece of the present invention comprises a labeling body-containing part that holds the labeling body in an elutable manner, and it is more preferable that the immunochromatographic test piece comprises the labeling body-containing part upstream of the capturing body immobilization part. In these cases, when the sample comes into contact with the labeling body-containing part, the labeling body is eluted, and when the target substance is present in the sample, a complex (immune complex) in which the target substance is bound to the labeling body is formed by an antigen-antibody reaction between the target substance and the detection body constituting the labeling body.

As the labeling body-containing part, even if the blood cell removal pad includes the labeling body-containing part and a part of the blood cell removal pad also serves as the labeling body-containing part as in Examples 1 to 3 described below, or even if the labeling body is held in an elutable manner in a separate water-absorbent material as a labeling body-containing pad and this is laminated on the upper surface or the lower surface of the blood cell removal pad, it may be provided at a position different from this, downstream of the blood cell removal pad. The water-absorbent material is preferably a porous material, and examples thereof include the porous membranes mentioned for the blood cell removal pad. Also, when provided at a position different from the blood cell removal pad, the labeling body may be held in the matrix in an elutable manner to serve as the labeling body-containing part. Furthermore, when it is laminated on the blood cell removal pad as a labeling body-containing pad, this is preferably at a position overlapping with the addition part; and when the labeling body-containing pad is laminated on the upper surface of the blood cell removal pad, the sample may be added from the upper surface of the labeling body-containing pad. Among these, when the immunochromatographic test piece of the present invention comprises the labeling body-containing part, it is preferable that the labeling body-containing, pad is laminated on the upper surface of the blood cell removal pad, or the blood cell removal pad includes the labeling body-containing part, and it is more preferable that the blood cell removal pad includes the labeling body-containing part.

The size of the labeling body holding part is not particularly limited, but when the matrix is in a strip shape, examples thereof include a range of a width of 1 mm to 10 mm and a length of 3 mm to 30 mm. When the labeling body holding part is the labeling body-containing pad separate from the blood cell removal pad, examples of the thickness thereof include a range of 0.3 mm to 2 mm. Also, when the blood cell removal pad includes the labeling body-containing part, in the blood cell removal pad, the position of the labeling body-containing part is preferably at a position overlapping with the addition part (more preferably, the addition part also serves as the labeling body-containing part); in this case, it is preferably on an upstream side in the blood cell removal pad, more preferably between the inside of the blood cell removal pad and within 15 mm from an upstream end of the blood cell removal pad, further preferably within an area of 50% on the upstream end side among the total area of the blood cell removal pad, and even more preferably within an area of 32%. Also, it is preferable that the labeling body-containing part at this time is applied or impregnated from the upper surface of the blood cell removal pad. Note that the shape of the labeling body holding part is not particularly limited, and can be, for example, a dot shape or a line shape.

A method for causing the labeling body-containing part to hold the labeling body is not particularly limited as long as it is a method in which the labeling body becomes elutable into the sample when the sample is developed; examples thereof include a method of applying or impregnating a solution containing the labeling body into the blood cell removal pad or the labeling body-containing pad or the matrix described above and then drying it. Examples of the solution for containing the labeling body include buffer solutions, and examples of the buffer solutions include buffer solutions adjustable to a pH suitable for a target antigen-antibody reaction, and more specifically, phosphate buffer solution, Tris buffer solution, Good's buffer solution, borate buffer solution, and the like. The amount of the labeling body to be held in the labeling body-containing part can be appropriately adjusted by the amount of the sample, sensitivity of the detection body contained in the labeling body to the target substance, the purpose of detection, and the like.

### (Developing Solution Supply Part)

It is preferable that the immunochromatographic test piece of the present invention comprises a developing solution supply part (developing solution supply part 7 in Figs. 2 to 6) for supplying a developing solution for carrying out immunochromatography. To the developing solution supply part, the developing solution may be dropped and supplied, or supplied from a device or the like containing the developing solution such as a developing solution tank described below. The developing solution supply part according to the present invention is preferably disposed most upstream of the matrix.

The developing solution supply part may be integral with the matrix, but is preferably a member made of a separate water-absorbent material like the developing solution pad 7 (Figs. 4 to 6). A base material of such a developing solution supply part is not particularly limited, and examples thereof include water-absorbent materials such as cellulose filter paper, nonwoven fabric, cloth, and cellulose acetate membrane.

The size of the developing solution supply part is not particularly limited, but when the matrix is in a strip shape, examples thereof include a range of a width of 1 mm to 10 mm and a length of 10 mm to 50 mm, and examples of the thickness of the developing solution pad include a range of 0.5 mm to 2 mm.

### (Development Confirmation Zone)

Furthermore, the immunochromatographic test piece of the present invention may further comprise a development confirmation zone (development confirmation zone 11 in Figs. 4 to 5) in which a substance capable of capturing the labeling body is immobilized to the matrix.

The development confirmation zone according to the present invention functions as a control zone for confirming success or failure of development of the developing solution in the immunochromatographic test piece of the present invention and determining whether the test is valid. The development confirmation zone according to the present invention is disposed downstream of the labeling body-containing part. Also, the development confirmation zone according to the present invention is preferably disposed downstream of the capturing body immobilization part.

In the present invention, the "substance capable of capturing the labeling body (hereinafter, optionally referred to as 'control body')" is a substance capable of binding to the labeling body and capable of capturing the labeling body. Such a control body is preferably a substance that does not bind to the target substance, and is typically an antibody or antigen capable of binding to the labeling substance or the detection body contained in the labeling body. As such a control body, for example, when the labeling substance contained in the labeling body is an enzyme, an anti-enzyme antibody can be used; and when the detection body contained in the labeling body is a rabbit polyclonal antibody specific to the target substance, an anti-rabbit antibody can be used.

Note that in Figs. 4 to 5, the development confirmation zone 11 is formed in a line shape, but the shape of the development confirmation zone in the present invention is not limited thereto, and may be an arbitrary shape such as a circle or a polygon. Among these shapes, the shape of the development confirmation zone according to the present invention is preferably a line shape, and when the matrix is in a strip shape, for example, a line shape with a width of 3 mm to 10 mm and a length of 0.5 mm to 3 mm is more preferable.

A method for immobilizing the control body to the development confirmation zone according to the present invention is not particularly limited, and a conventionally known method or a method according thereto can be appropriately adopted; methods similar to those cited as the method for immobilizing the capturing body to the capturing body immobilization part described above are cited. The amount of the control body to be immobilized to the development confirmation zone according to the present invention can be appropriately adjusted by the form of the sample, the type of the labeling substance or antibody contained in the labeling body, the purpose of detection, and the like.

In the immunochromatographic test piece of the present invention, when the labeling body (a labeling body that was not captured in the capturing body immobilization part when the development confirmation zone is disposed downstream of the capturing body immobilization part) moves to the development confirmation zone, the labeling body is captured by the control body immobilized to the development confirmation zone, and the labeling substance constituting the labeling body accumulates in the development confirmation zone. As a result, since the development confirmation zone shows a signal due to accumulation of the labeling substance, by detecting this, it is possible to confirm that the labeling body has been developed to the development confirmation zone.

### (Absorption Zone)

The immunochromatographic test piece of the present invention may further comprise an absorption zone (absorption zone 9 in Figs. 2 to 6) as a member having a function of absorbing the sample and the developing solution that have moved through the matrix which is a chromatography medium, the labeling body that was not captured in the capturing body immobilization part or the development confirmation zone, and the like. The absorption zone according to the present invention is preferably disposed most downstream of the matrix.

The absorption zone may be integral with the matrix, but is preferably a member made of a separate water-absorbent material like the absorption pad 9 (Figs. 4 to 6). A base material of such an absorption zone is not particularly limited, and examples thereof include water-absorbent materials such as cellulose filter paper; nonwoven fabric; cloth; cellulose acetate membrane; and synthetic fiber (synthetic fibers such as silica gel, polyethylene terephthalate, polyvinyl alcohol, and cellulose) paper.

The size of the absorption zone is not particularly limited, but when the matrix is in a strip shape, examples thereof include a range of a width of 1 mm to 10 mm and a length of 4 mm to 6 mm, and examples of the thickness of the absorption pad include a range of 0.5 mm to 2 mm.

### (Substrate Zone, Substrate Addition Part)

As for the immunochromatographic test piece of the present invention, when the labeling substance contained in the labeling body is an enzyme, the developing solution may contain a substrate in advance; however, it is preferable that the immunochromatographic test piece further comprises, on the matrix, a substrate zone (substrate zone 10 in Figs. 2 to 4) that holds a substrate of the enzyme in an elutable manner, or an addition part (substrate addition part) for adding the substrate of the enzyme during or after development of the developing solution; in this case, it is preferable that the matrix includes the substrate zone or the substrate addition part. The substrate zone or the substrate addition part according to the present invention is disposed upstream of the capturing body immobilization part. Also, the substrate zone or the substrate addition part according to the present invention is preferably disposed upstream of the labeling body-containing part.

A method for causing the substrate zone according to the present invention to hold the substrate is not particularly limited as long as it is a method in which the substrate becomes elutable into the sample or the developing solution when they are developed; methods similar to those cited as the method for causing the labeling body-containing part to hold the labeling body described above are cited. Also, a method for adding the substrate to the substrate addition part according to the present invention is not particularly limited. The amount of the substrate to be held in the substrate zone according to the present invention or the amount of the substrate to be added to the substrate addition part can be appropriately adjusted by the types of the enzyme and substrate, and the like.

Further, the immunochromatographic test piece of the present invention may further comprise a support (not shown; for example, an adhesive sheet made of plastic, metal, paper, or the like) for supporting the matrix and each pad, as necessary. Also, an adhesive tape or the like for adhering and fixing each member may be further included as long as the effects of the present invention are not inhibited.

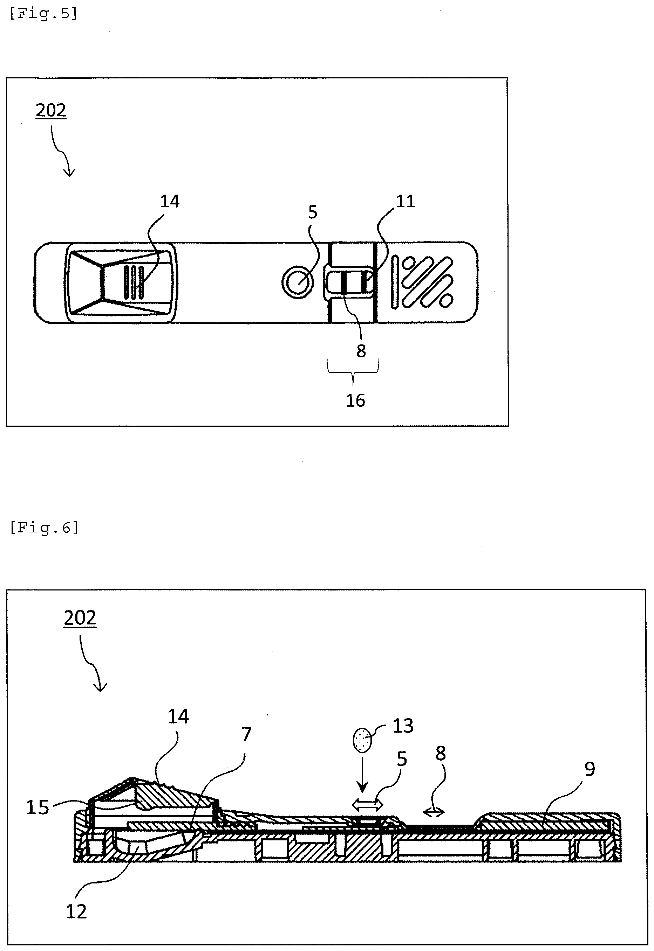

The immunochromatographic apparatus of the present invention only needs to comprise the immunochromatographic test piece of the present invention; for example, it may further have a developing solution tank (developing solution tank 12 in Figs. 4 to 6) for storing the developing solution upstream of the matrix of the immunochromatographic test piece, a protrusion part (protrusion part 15 in Fig. 6) for supplying the developing solution in the developing solution tank to the matrix through the developing solution pad, a push-in part (push-in part 14 in Figs. 5 to 6) for pressurizing and moving the protrusion part, and the like; and it may comprise a case or the like that accommodates the immunochromatographic test piece of the present invention and is provided with a determination window (determination window 16 in Fig. 5) for checking the detection zone and the development confirmation zone.

In the immunochromatographic test piece of the present invention, the arrangement of each member is as described above and is not particularly limited; however, in the case where the labeling substance contained in the labeling body is an enzyme and the purpose is to carry out enzyme immunoassay, from the viewpoint of excellent accuracy thereof, it is preferable that the immunochromatographic test piece is in a strip shape, and the developing solution supply part, the blood cell removal pad and the waterproof layer, and the capturing body immobilization part are disposed in this order from upstream; and it is more preferable that the blood cell removal pad comprises the labeling body-containing part, and/or the substrate zone or the substrate addition part is disposed between the developing solution supply part and the blood cell removal pad.

### <Target Substance Detection Method>

The present invention relates to a method for detecting a target substance in a sample containing blood cells by immunochromatography, and also provides a target substance detection method comprising a step of adding the sample to the addition part (step 1) and a step of causing the matrix to develop a developing solution (step 2) in the immunochromatographic test piece of the present invention, wherein the sample and the developing solution are brought into contact at the protruding portion of the blood cell removal pad.

As one embodiment of the target substance detection method of the present invention, a target substance detection method (immunochromatographic method) using the immunochromatographic test piece described in Figs. 1 to 4 will be described below as an example.

In the target substance detection method of the present invention, first, a sample 13 is dropped or applied to the addition part 5 to be added thereto and received (step 1). It is preferable that the sample 13 is first brought into contact with the labeling body; in this case, the sample 13 may be mixed with the labeling body in advance and then added to the addition part 5, but it is more preferable to add the sample 13 to the addition part 5 so as to come into direct contact with the labeling body, and for example, it is further preferable that the addition part 5 also serves as the labeling body-containing part. When the addition part 5 also serves as the labeling body-containing part, by bringing the sample 13 into contact with the labeling body held in the labeling body-containing part, the labeling body is eluted, and when the target substance is present in the sample 13, a complex (immune complex) of the target substance and the labeling body is formed by an antigen-antibody reaction between the target substance and the detection body constituting the labeling body (labeling step).

Also, to the matrix 1, the developing solution is supplied upstream of the addition part 5, preferably from the developing solution supply part 7, simultaneously with or separately from the sample 13 (preferably after addition of the sample 13) (step 2). For example, as shown in the immunochromatographic apparatus 202 of Figs. 5 to 6, by pressurizing the push-in part 14 to move the protrusion part 15, the developing solution pad 7 is inserted into the developing solution tank 12, and the developing solution can be supplied to the matrix 1 through the developing solution pad 7. Examples of the developing solution include buffer solutions adjusted to a pH suitable for a target antigen-antibody reaction, and more specific examples of the buffer solutions include phosphate buffer solution, Tris buffer solution, Good's buffer solution, borate buffer solution, and 2-amino-2-methyl-1-propanol (AMP) buffer solution. Also, one kind or two or more kinds among surfactants, blood coagulation inhibitors, and the like may be contained.

Also, when the labeling substance contained in the labeling body is an enzyme, it is preferable that the immunochromatographic test piece of the present invention further comprises the substrate zone 10. When the immunochromatographic test piece further comprises the substrate zone 10, when the developing solution passes through the substrate zone 10, the substrate is eluted into the developing solution, and the developing solution containing the substrate flows. Note that as for the substrate, a substrate solution may be dropped or applied to the substrate addition part simultaneously with or separately from the developing solution. Examples of the solvent of the substrate in this case include the buffer solutions described above.

The developing solution supplied to the matrix 1 moves in the matrix 1 in the development direction 4 of the developing solution by capillary action. Also, the sample 13 received in the addition part 5 moves in the blood cell removal pad 2 in the development direction 4 of the developing solution by capillary action. At this time, the sample 13 does not migrate in a vertical direction from the blood cell removal pad 2 to the matrix 1 due to the waterproof layer 3.

Regarding the sample 13 received in the addition part 5, after blood cells are removed by moving in the blood cell removal pad 2 in the development direction 4 of the developing solution, remaining components (for example, plasma and serum) containing the target substance (preferably the complex of the target substance and the labeling body) come into contact with the matrix 1 at the protruding portion 6, and come into contact with the developing solution moving in the matrix 1. The developing solution and the sample (sample obtained by removing blood cells from the sample 13) move in the matrix 1 in the development direction 4 of the developing solution, and reach the capturing body immobilization part 8 (detection zone 8) in the aspects described in Figs. 2 to 6. Thereby, in the capturing body immobilization part 8, the immune complex is captured by the capturing body immobilized to the capturing body immobilization part 8 via the target substance (capturing step). As a result of the immune complex being captured in the capturing body immobilization part 8, the capturing body immobilization part 8 shows coloration or the like by the labeling substance constituting the labeling body; therefore, this can be detected as a signal (detection step), and can be quantified as necessary. Also, when the labeling substance is an enzyme, in the aspects described in Figs. 2 to 6, since the developing solution containing the substrate moves to the capturing body immobilization part 8, a signal generated by an enzyme-substrate reaction can be detected.

In the target substance detection method of the present invention, the capturing step and the detection step may be simultaneous, or the detection step may be carried out after the capturing step; for example, a signal such as coloration generated simultaneously with the capturing step may be detected with the naked eye, or after capturing the complex in the capturing step, a detection step of performing a treatment according to the labeling substance contained in the complex (addition of substrate, observation by a fluorescence microscope, detection by a fluorometer, detection by a scintillation counter, etc.) may be carried out.

When the target substance is not contained in the sample, the labeling body is not immobilized to the capturing body immobilization part 8 and moves further downstream; therefore, a signal is not detected in the capturing body immobilization part 8. Also, when the concentration of the target substance in the sample is low, the amount of the labeling substance accumulated in the capturing body immobilization part 8 decreases and the signal intensity becomes weak; thus, quantitation of the target substance is possible by the intensity thereof.

Also, in the aspects described in Figs. 4 to 6, the labeling body (a labeling body that was not captured in the capturing body immobilization part 8 when the development confirmation zone 11 is disposed downstream of the capturing body immobilization part 8) moves further downstream and reaches the development confirmation zone 11 as well. Thereby, in the development confirmation zone 11, the labeling body is captured and detected by the control body immobilized to the development confirmation zone 11 (confirmation step). Note that in the aspects described in Figs. 4 to 6, the confirmation step is performed after the capturing step, but the capturing step and the confirmation step may be independent of each other, may be simultaneous at the same position, or the capturing step may be performed after the confirmation step. As a result of the labeling body being captured in the development confirmation zone 11, the development confirmation zone 11 shows coloration or the like by the labeling substance constituting the labeling body; therefore, this can be detected as a signal, and by quantifying it as necessary, it is possible to determine that the sample has moved normally on the immunochromatographic test piece, that is, to determine whether the test is valid. The criterion for the determination can be appropriately set according to the purpose of detection, the amount of the target substance, and the like. In the aspects described in Figs. 2 to 6, the developing solution is finally absorbed by the absorption zone 9 (absorption pad 9) downstream thereof.

### <Immunochromatographic Kit>

The immunochromatographic kit of the present invention includes at least the immunochromatographic test piece of the present invention. The immunochromatographic test piece may be included as part of the immunochromatographic apparatus of the present invention.

Also, the immunochromatographic kit of the present invention may further include at least one kind selected from the group consisting of a diluent of the sample and a developing solution, as necessary. Furthermore, the labeling body, the capturing body, the control body, or antibodies, antigens, labeling substances, and the like constituting these can also be combined. Also, for example, when the labeling substance is an enzyme, a substrate necessary for detection of the label, a reaction stop solution, and the like can also be included. Also, the immunochromatographic kit of the present invention can further include an instruction manual for the kit.

The immunochromatographic test piece, the immunochromatographic apparatus, the target substance detection method, and the immunochromatographic kit of the present invention can be used, for example, as research supplies, and can also be used as in vitro diagnostic reagents for detection of the target substance which serves as a basis for diagnosis of diseases related to the target substance.

### [Examples]

Hereinafter, the present invention will be described more specifically based on examples and comparative examples, but the present invention is not limited to the following examples. In particular, each antibody, antigen, their amounts, reaction conditions, and the like can be appropriately adjusted.

### (1) Production of Immunochromatographic Apparatus

### (Examples 1 to 3)

An immunochromatographic apparatus having the configuration shown in Fig. 4 was produced. First, an immunochromatographic test piece was produced.

### (Matrix, Waterproof Layer)

A nitrocellulose membrane (porous membrane, manufactured by Merck or manufactured by Sartorius) having a width of 3.65 mm and a length of 50 mm was used as the matrix 1, and one short side of the matrix 1 was defined as a left end. Also, a waterproof tape (polyester film, Scotch Tape (manufactured by Kuramoto Sangyo Co., Ltd.)) having a width of 3.65 mm, a length of 13 mm, and a thickness of 0.025 mm was used as the waterproof layer 3, and one short side of the waterproof layer 3 was defined as a left end. The waterproof layer 3 was pasted so that the left end of the waterproof layer 3 was aligned at a position 15 mm from the left end of the matrix 1.

### (Development Confirmation Zone, Detection Zone)

At a position 12 mm from a right end of the matrix 1, 0.54 µL of a control body solution containing 21.4 µg/mL of an anti-ALP antibody was spotted in a line shape and dried to form the development confirmation zone 11. Also, at a position 15 mm from the right end of the matrix 1, 0.54 µL of an antibody or antigen solution against a target substance (antigen A or antibody B) was spotted in a line shape and dried to form the detection zone 8 (capturing body immobilization part 8).

### (Substrate Zone)

At a position 47 mm from the right end of the matrix 1, a substrate (5-bromo-4-chloro-3-indolyl phosphate (BCIP)) solution was spotted in a line shape so that the substrate amount became 27.8 µg and dried to form the substrate zone 10.

### (Blood Cell Removal Pad, Addition Part)

With one short side of each porous membrane shown in Table 1 below having a width of 3.65 mm and a length of 15.0 mm defined as a left end, 3.04 µL of a labeling body solution containing a labeling body obtained by ALP-labeling an antibody or antigen against the target substance (antigen A or antibody B) was spotted and dried on an upper surface at a position 4.75 mm from the left end to serve as the addition part 5 and the labeling body-containing part 5, and the blood cell removal pad 2 including the addition part 5 and the labeling body-containing part 5 was produced. The obtained blood cell removal pad 2 was pasted on the waterproof layer 3 on the matrix 1 with a double-sided tape (base material: nonwoven fabric, width 3.65 mm, length 10 mm, manufactured by 3M Company) by aligning the left end of the blood cell removal pad 2 and the left end of the waterproof layer 3. Thereby, the blood cell removal pad 2 has a configuration in which it protrudes 2.0 mm from a right end of the waterproof layer 3 (waterproof tape) and contacts the matrix 1 only at the protruding portion.

**[Table 1]**

| | Material | Thickness | Flow rate | Water absorption amount | Particle retention capacity | Product name / Distributor name |
|---|---|---|---|---|---|---|
| | | [µm] | [s/4cm] | [mg/cm²] | [µm] | |
| Example 1 | Glass fiber | 484 | 45 | 47 | 2.3 | Fusion5 / Cytiva |
| Example 2 | Glass fiber | 862 | 22 | 88 | 3 | VF2 / Cytiva |
| Example 3 | Glass fiber | 924 | 30 | 116 | 2.7 | GF/D / Cytiva |

Table 1 shows the thickness, flow rate, water absorption amount, particle retention capacity, and product name and distributor name of the porous membrane used as the blood cell removal pad in each Example (and Comparative Example below) . In Table 1, "Thickness" indicates a thickness (µm) measured by a caliper under atmospheric pressure at an air temperature of 20°C and a humidity of 50%; "Flow rate" indicates a speed (s/4 cm) at which water passes through the inside of the porous membrane from the lower surface toward the upper surface direction under atmospheric pressure at an air temperature of 20°C and a humidity of 50%; "Water absorption amount" indicates an amount of water (g/cm²) absorbed in 1 cm² of the porous membrane under atmospheric pressure at an air temperature of 20 ° C and a humidity of 50%; and "Particle retention capacity" indicates a value obtained by measuring a particle diameter (µm) of particles 98% or more of which are retained in the porous membrane by flow cytometry or the like.

Next, the developing solution pad 7 (porous cellulose membrane) and the absorption pad 9 (synthetic fiber paper made of silica gel, polyethylene terephthalate, polyvinyl alcohol, and cellulose) were pasted to the matrix 1 as shown in Fig. 4 to obtain an immunochromatographic test piece. The produced immunochromatographic test piece was fixed to a plastic case having the developing solution tank 12 filled with the developing solution, the push-in part 14, the protrusion part 15, and the determination window 16 as shown in Figs. 4 to 6 to obtain an immunochromatographic apparatus.

### (Comparative Examples 1 to 3)

Immunochromatographic apparatuses of Comparative Examples 1 to 3 were produced in the same manner as in Examples 1 to 3, respectively, except that the waterproof layer 3 (waterproof tape) was not pasted on the matrix 1, and the left end of the blood cell removal pad 2 was aligned at a position 15 mm from the left end of the matrix 1 so that the blood cell removal pad 2 and the matrix 1 were in direct contact, and the left end was pasted with a double-sided tape (base material: transparent polyester film, width 3.65 mm, length 5 mm, manufactured by Kuramoto Sangyo Co., Ltd.). In Example 1 and Comparative Example 1, Example 2 and Comparative Example 2, and Example 3 and Comparative Example 3, respectively, the base materials and their configurations are common to each other except for the presence or absence of the waterproof layer. In the immunochromatographic apparatuses obtained in Comparative Examples 1 to 3, the blood cell removal pad 2 has a configuration in which almost the entire surface is in direct contact with the matrix 1.

### (2) Evaluation 1 of Immunochromatographic Apparatus

For two types of target substances (antigen A and antibody B) for which a test with a whole blood specimen is assumed, a detection test was performed using each immunochromatographic apparatus produced in Examples 1 and 3 above. Note that in the immunochromatographic apparatus used in this test, as the nitrocellulose membrane, one manufactured by Merck was used for antigen A, and those manufactured by Sartorius were used for antibody B in all cases. First, antigen A was added to a whole blood specimen (healthy person whole blood specimen) so as to be 100 ng/mL to prepare a pseudo whole blood positive specimen. Also, an antibody B positive plasma specimen was diluted 20-fold or 200-fold with a whole blood specimen (healthy person whole blood specimen) to prepare a pseudo whole blood positive specimen. Next, each pseudo whole blood positive specimen was dropped onto the addition part 5 of each immunochromatographic apparatus. The dropping amount was 25 µL for Example 1 and 50 µL for Example 3. Immediately after dropping, the push-in part 14 was pressed to develop the developing solution, and color development in the determination window (detection zone 8 and development confirmation zone 11) 15 minutes after pressing was visually confirmed. Note that since each pseudo whole blood positive specimen is used as a specimen in this test, when correctly detected, all show positive (blue lines are observed in the detection zone 8 and the development confirmation zone 11) .

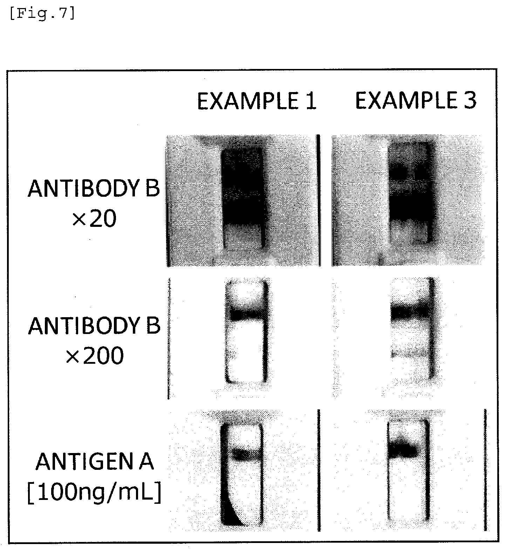

The appearance of the determination window (detection zone 8 and development confirmation zone 11) at the time of visual confirmation is shown in Fig. 7. In Fig. 7, in each determination window, the upper line is the line of the development confirmation zone 11 (reference line), and the lower line is the line of the detection zone 8 (test line) . As shown in Fig. 7, in the immunochromatographic apparatus comprising the immunochromatographic test piece of the present invention (Examples 1 and 3), two lines, that is, respective blue to pale blue lines of the detection zone 8 and the development confirmation zone 11 could be confirmed, and it was confirmed that detection of the target substance from the whole blood specimen could be easily performed.

### (3) Evaluation 2 of Immunochromatographic Apparatus

Using a healthy person whole blood specimen (negative specimen) as a whole blood specimen, a comparative test of each immunochromatographic apparatus produced in Examples 1 to 3 and Comparative Examples 1 to 3 above was performed. Note that in the immunochromatographic apparatus used in this test, as the nitrocellulose membranes, those manufactured by Merck were all used. The test was performed in the same manner as in (2) above except that for each immunochromatographic apparatus, a healthy person whole blood specimen (negative specimen) was used as the whole blood specimen, and the dropping amount dropped onto the addition part 5 was set to 25 or 30 µL (46 or 55 µL/cm²) for Example 1 and Comparative Example 1, 35, 40, or 45 µL (64, 73, or 82 µL/cm²) for Example 2 and Comparative Example 2, and 50, 55, or 60 µL (91, 100, or 110 µL/cm²) for Example 3 and Comparative Example 3; and color development in the determination window (detection zone 8 and development confirmation zone 11) 15 minutes after pressing the push-in part 14 was visually confirmed. However, since development was slow in Example 3, confirmation was performed 30 minutes after pressing the push-in part 14. Note that since a negative specimen is used as a specimen in this test, when correctly detected, all show negative (a blue line (reference line) is observed in the development confirmation zone 11, but color development is not confirmed in the detection zone 8).

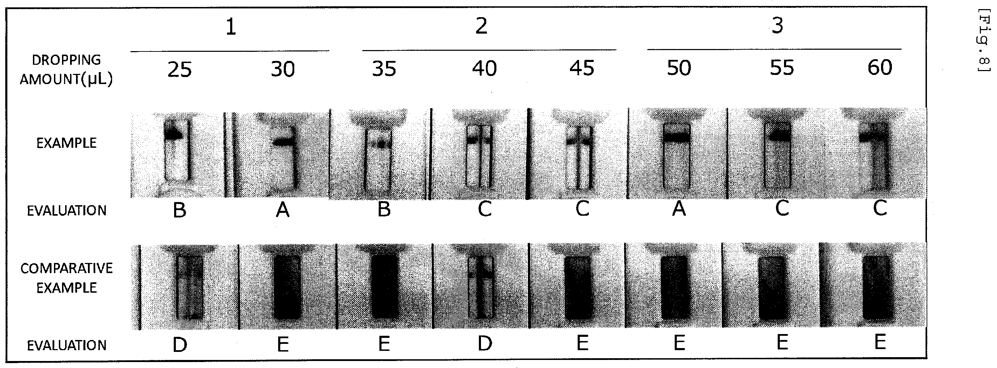

The appearance of the determination window (detection zone 8 and development confirmation zone 11) at the time of visual confirmation is shown in Fig. 8. In Fig. 8, in each determination window, the upper line is the line of the development confirmation zone 11 (reference line), and the lower line is the line of the detection zone 8 (test line) . Also, the appearance of the determination window was visually evaluated by the following criteria:
A: Reference line can be clearly confirmed. No coloration other than reference line in determination window
B: 2/3 or more of reference line can be confirmed. However, unnecessary blue vertical streaks exist in determination window, or reference line color intensity is weak
C: 2/3 or more of reference line can be confirmed. However, red vertical streaks derived from blood exist in determination window
D: Confirmed reference line is less than 2/3
E: Entire surface of determination window is colored red, and color development of reference line is difficult to confirm

In the evaluation, A is the highest evaluation, and if it is C or higher, it is practicable as an immunochromatographic apparatus. The evaluation results are collectively shown in Fig. 8.

As shown in Fig. 8, in the immunochromatographic apparatus comprising the immunochromatographic test piece of the present invention (Examples 1 to 3, upper row of Fig. 8), all evaluations were C or higher, and it was confirmed that confirmation of the determination window could be clearly performed even using the whole blood specimen. On the other hand, in the immunochromatographic apparatus comprising the immunochromatographic test piece in which the waterproof layer was not installed (Comparative Examples 1 to 3, lower row of Fig. 8), all evaluations were D or lower, and the reference line in the determination window was unclear or could not be confirmed; thus, it was confirmed that it is difficult to apply to a test using a whole blood specimen.

### (4) Study of Immunochromatographic Apparatus

### (4-1) Production of Immunochromatographic Apparatus

### (Example 1-1, Example 3-1)

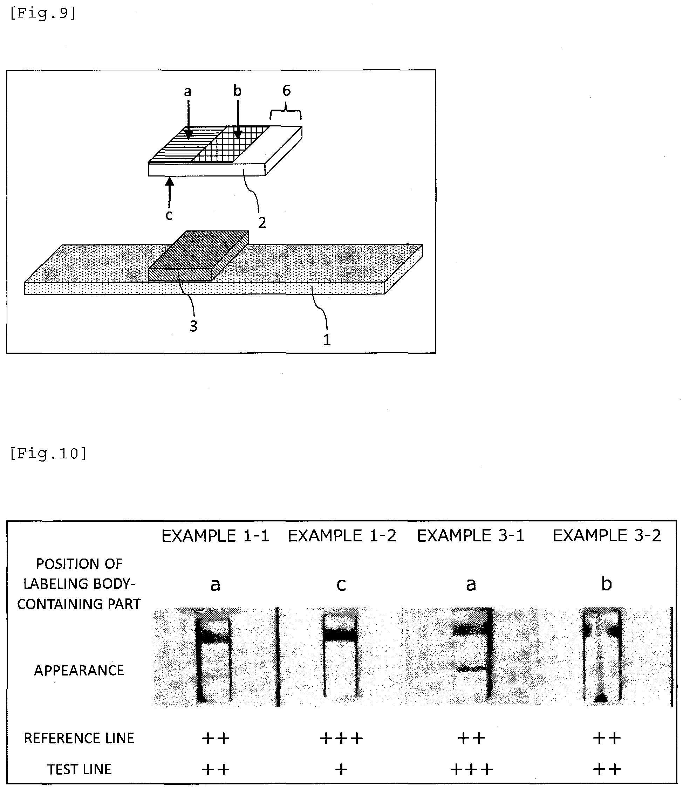

In the blood cell removal pad comprising the labeling body-containing part, the position of the labeling body-containing part was studied. First, in the same manner as in Examples 1 and 3 except that a labeling body obtained by ALP-labeling an antibody against antigen C (target substance) described below was used as the labeling body, with one short side of each porous membrane having a width of 3.65 mm and a length of 15.0 mm defined as a left end, a labeling body solution was spotted and dried on the upper surface at a position 4.75 mm from the left end and at the center in the width direction (within a horizontal line portion shown by 'a' in Fig. 9) to serve as the labeling body-containing part, and the blood cell removal pad 2 including the labeling body-containing part was produced. Also, using the obtained blood cell removal pad 2, in the same manner as in Examples 1 and 3 except that an antibody against antigen C was used for the detection zone 8 (capturing body immobilization part 8), the blood cell removal pad 2 was pasted on the waterproof layer 3 on the matrix 1 with a double-sided tape so that its left end and the left end of the waterproof layer 3 were aligned, and immunochromatographic apparatuses of Example 1-1 and Example 3-1 were produced respectively. Except that the position of each labeling body-containing part was controlled as described above, Example 1-1 is common to Example 1, and Example 3-1 is common to Example 3 in terms of the base materials of each immunochromatographic apparatus and their configurations. Note that as the nitrocellulose membranes, those manufactured by Merck were all used.

### (Example 1-2)

An immunochromatographic apparatus was produced by pasting the obtained blood cell removal pad 2 on the waterproof layer 3 on the matrix 1 in the same manner as in Example 1-1 except that it was turned upside down so that the surface on which the labeling body solution was spotted and dried was within the position 'c' in Fig. 9. That is, on the matrix 1, the labeling body-containing part is arranged such that the content of the labeling body is larger on the waterproof layer 3 side of the lower surface of the blood cell removal pad 2. Except that the position of the labeling body-containing part was controlled as described above, the base materials of the immunochromatographic apparatus and their configurations are common to Example 1 and Example 1-1.

### (Example 3-2)

An immunochromatographic apparatus was produced by pasting the obtained blood cell removal pad 2 on the waterproof layer 3 on the matrix 1 in the same manner as in Example 3-1 except that the left and right were reversed so that the position of the labeling body-containing part was within the position 'b' in Fig. 9. That is, on the matrix 1, the labeling body-containing part is disposed at a position 10.25 mm from the left end of the blood cell removal pad 2 and at the center in the width direction (within a grid line portion shown by 'b' in Fig. 9). Except that the position of the labeling body-containing part was controlled as described above, the base materials of the immunochromatographic apparatus and their configurations are common to Example 3 and Example 3-1.

### (4-2) Evaluation 3 of Immunochromatographic Apparatus

Using each immunochromatographic apparatus produced in (4-1) above, a detection test was performed for a target substance (antigen C) for which a test with a whole blood specimen is assumed. First, antigen C was added to a whole blood specimen (healthy person whole blood specimen) so as to be 10 ng/mL to prepare a pseudo whole bloodpositive specimen. Next, the prepared pseudo whole blood positive specimen was dropped onto the addition part 5 (between the left end and 5 mm on the upper surface of the blood cell removal pad 2, regardless of the position of the labeling body-containing part) of each immuno chromato graphic apparatus. The dropping amount was 25 µL for Example 1-1 and Example 1-2, and 45 µL for Example 3-1 and Example 3-2. Immediately after dropping, the push-in part 14 was pressed to develop the developing solution, and color development in the determination window (detection zone 8 and development confirmation zone 11) 15 minutes after pressing was visually confirmed. Note that since a pseudo whole blood positive specimen is used as a specimen in this test, when correctly detected, all show positive (blue lines are observed in the detection zone 8 and the development confirmation zone 11).

The appearance of the determination window (detection zone 8 and development confirmation zone 11) at the time of visual confirmation is shown in Fig. 10. In Fig. 10, in each determination window, the upper line is the line of the development confirmation zone 11 (reference line), and the lower line is the line of the detection zone 8 (test line) . Also, the reference line and the test line were visually evaluated by the following criteria:
+++: Line color is blue with higher saturation than pale blue
++: Line color is pale blue
+: Pale blue line is observed, but minimum color intensity visible in photograph
-: Color development of test line is difficult to conf irm or confirmed test line is less than 2/3
Undeterminable: Color development of reference line is difficult to confirm or confirmed reference line is less than 2/3 (test invalid)
In the evaluation, +++ is the highest evaluation, and if it is + or higher, it is practicable as an immunochromatographic apparatus. The evaluation results are collectively shown in Fig. 10.

As shown in Fig. 10, in the immunochromatographic apparatus comprising the immunochromatographic test piece of the present invention, regardless of the position of the labeling body-containing part, all evaluations were + or higher, and the reference line and the test line could be confirmed. Among them, as the position of the labeling body-containing part in the blood cell removal pad, the position 'a' in Fig. 9, that is, on the upstream side in the development direction of the developing solution and overlapping with the addition part to which the specimen is added, showed high evaluation in both the reference line and the test line, and tended to be more preferable.

### [Industrial Applicability]

As described above, according to the present invention, it becomes possible to provide an immunochromatographic test piece capable of easily detecting a target substance in a sample containing blood cells such as a whole blood specimen and also allowing enzyme immunoassay to be performed with high accuracy, an immunochromatographic apparatus comprising the same, an immunochromatographic kit including at least any of these, and a method for detecting a target substance in a sample containing blood cells using at least any of these.

### [Reference Signs List]

101, 102, 103: Immunochromatographic test piece
201, 202: Immunochromatographic apparatus
1: Matrix
2: Blood cell removal pad
3: Waterproof layer
4: Development direction of developing solution
5: Addition part (Labeling body-containing part in Examples 1 to 3, 1-1, 3-1)
6: Protruding portion
7: Developing solution supply part, Developing solution pad
8: Capturing body immobilization part (Detection zone)
9: Absorption zone, Absorption pad
10: Substrate zone
11: Development confirmation zone
12: Developing solution tank
13: Sample
14: Push-in part
15: Protrusion part
16: Determination window

## Claims

1. An immunochromatographic test piece for use in detecting a target substance in a sample containing blood cells by immunochromatography, comprising:
a matrix for developing a developing solution;
a blood cell removal pad including an addition part of the sample, laminated on the matrix; and
a waterproof layer laminated between the matrix and the blood cell removal pad,
wherein the blood cell removal pad has a protruding portion protruding onto the matrix further in a development direction of the developing solution than the waterproof layer.

2. The immunochromatographic test piece according to claim 1, wherein a length of the protruding portion of the blood cell removal pad is from 1 mm to 4 mm.

3. The immunochromatographic test piece according to claim 1, wherein the blood cell removal pad is a glass fiber membrane.

4. The immunochromatographic test piece according to claim 1, comprising a capturing body immobilization part in which a capturing body capable of capturing the target substance is immobilized, down stream of the blood cell removal pad on the matrix.

5. The immunochromatographic test piece according to claim1, comprising a labeling body- containing part that holds, in an elutable manner, a labeling body in which a labeling substance is bound to an antibody or antigen capable of binding to the target substance.

6. The immunochromatographic test piece according to claim 5, wherein the labeling substance is an enzyme.

7. The immunochromatographic test piece according to claim 6, wherein the developing solution contains a substrate of the enzyme, or the immunochromatographic test piece comprises a substrate zone that holds a substrate of the enzyme in an elutable manner upstream of the capturing body immobilization part on the matrix, or comprises an addition part of a substrate of the enzyme on the matrix.

8. The immunochromatographic test piece according to claim 5, wherein a labeling body-containing pad including the labeling body-containing part is laminated on the blood cell removal pad, or the blood cell removal pad comprises the labeling body-containing part.

9. An immunochromatographic apparatus used for detecting a target substance in a sample containing blood cells by immunochromatography, comprising the immunochromatographic test piece according to any one of claims 1 to 8.

10. An immunochromatographic kit used for detecting a target substance in a sample containing blood cells by immunochromatography, including the immunochromatographic test piece according to any one of claims 1 to 8.

11. A method for detecting a target substance in a sample containing blood cells by immunochromatography, comprising the steps of:
in an immunochromatographic test piece comprising: a matrix for developing a developing solution; a blood cell removal pad including an addition part of the sample, laminated on the matrix; and a waterproof layer laminated between the matrix and the blood cell removal pad, wherein the blood cell removal pad has a protruding portion protruding onto the matrix further in a development direction of the developing solution than the waterproof layer,
adding the sample to the addition part; and
causing the matrix to develop a developing solution,
wherein the sample and the developing solution are brought into contact at the protruding portion of the blood cell removal pad.
